# EUROPEAN PATENT SPECIFICATION

(11) **EP 1 612 283 B1**
(45) Date of publication and mention of the grant of the patent: **19.10.2011**
(21) Application number: 05447160.2
(22) Date of filing: 30.06.2005
(51) Int. Cl.: C12Q 1/68

(54) **Genotype specific detection of chlamydophila psittaci**
Genotyp spezifischer Detektion von Chlamydophila psittaci
Détection spécifique de gènotypes de Chlamydophilla psittaci

(30) Priority: 30.06.2004 US 584725 P
(43) Date of publication of application: 04.01.2006
(73) Proprietor: Universiteit Gent, 9000 Gent (BE)
(72) Inventor: Vanrompay, Daisy, 9860 Oosterzele, Balegem (BE)
(74) Representative: Bird, Ariane

(56) References cited:
- WO-A-03/044178
- US-B1- 6 261 769
- SUDLER CHRISTINE ET AL: "Molecular characterisation of chlamydial isolates from birds." VETERINARY MICROBIOLOGY. 5 MAR 2004, vol. 98, no. 3-4, 5 March 2004 (2004-03-05), pages 235-241, XP002347998 ISSN: 0378-1135
- VANROMPAY D ET AL: "Characterization of avian Chlamydia psittaci strains using omp1 restriction mapping and serovar-specific monoclonal antibodies" RESEARCH IN MICROBIOLOGY, vol. 148, no. 4, 1997, pages 327-333, XP000983466 ISSN: 0923-2508
- VANROMPAY DAISY ET AL: "Immunoblotting, ELISA and culture evidence for Chlamydiaceae in sows on 258 Belgian farms." VETERINARY MICROBIOLOGY. 26 MAR 2004, vol. 99, no. 1, 26 March 2004 (2004-03-26), pages 59-66, XP002347999 ISSN: 0378-1135
- BUSH R M ET AL: "Molecular evolution of the Chlamydiaceae." INTERNATIONAL JOURNAL OF SYSTEMATIC AND EVOLUTIONARY MICROBIOLOGY. JAN 2001, vol. 51, no. Pt 1, January 2001 (2001-01), pages 203-220, XP002348000 ISSN: 1466-5026
- DEGRAVES FRED J ET AL: "Quantitative detection of Chlamydia psittaci and C. pecorum by high-sensitivity real-time PCR reveals high prevalence of vaginal infection in cattle." JOURNAL OF CLINICAL MICROBIOLOGY. APR 2003, vol. 41, no. 4, April 2003 (2003-04), pages 1726-1729, XP002348069 ISSN: 0095-1137

### SEQUENCE LISTING

<110> Universiteit Gent vanrompay, Daisy
<120> Genotype Specific Detection of Chlamydophila psittaci
<130> R3380
<150> US 60/584,725
   <151> 2004-06-30
<160> 53
<170> PatentIn version 3.3
<210> 1
   <211> 30
   <212> DNA
   <213> artificial
<220>
   <223> Probe for Cp. psittaci genotype A
<400> 1
   ctaccgatct tccaacgcaa cttcctaacg 30
<210> 2
   <211> 33
   <212> DNA
   <213> artificial
<220>
   <223> Probe for Cp. psittaci genotype B
<400> 2
   tctaccgatc ttccaatgca acttcctaac gta 33
<210> 3
   <211> 31
   <212> DNA
   <213> artificial
<220>
   <223> Probe for Cp. psittaci genotype C
<400> 3
   tctgctgtta tgaacttgac cacatggaac c 31
<210> 4
   <211> 24
   <212> DNA
   <213> artificial
<220>
   <223> Probe for Cp. psittaci genotype D
<400> 4
   aggaaaggcc acaactgtcg acgg 24
<210> 5
   <211> 36
   <212> DNA
   <213> artificial
<220>
   <223> Probe for Cp. psittaci genotype E
<400> 5
   tactttgccc aataatggtg gtaaggatgt tctatc 36
<210> 6
   <211> 27
   <212> DNA
   <213> artificial
<220>
   <223> Probe for Cp. psittaci genotype F
<400> 6
   catcgctcaa cctaaattag ccgctgc 27
<210> 7
   <211> 34
   <212> DNA
   <213> artificial
<220>
   <223> competitor probe
<400> 7
   tctaccgatc cttccaacgc aacttcctaa cgta 34
<210> 8
   <211> 30
   <212> DNA
   <213> artificial
<220>
   <223> competitor probe
<400> 8
   ctaccgatct tccaatgcaa cttcctaacg 30
<210> 9
   <211> 36
   <212> DNA
   <213> artificial
<220>
   <223> competitor probe
<400> 9
   tgctttgccc aataatgctg gtaaggatgt tctatc 36
<210> 10
   <211> 33
   <212> DNA
   <213> artificial
<220>
   <223> competitor probe
<400> 10
   tctaccgagc ttccaatgca acttcctaac gta 33
<210> 11
   <211> 36
   <212> DNA
   <213> artificial
<220>
   <223> competitor probe
<400> 11
   tgctttgccc aataatagtg gtaaggatgt tctatc 36
<210> 12
   <211> 21
   <212> DNA
   <213> artificial
<220>
   <223> forward primer genotype A
<400> 12
   ggttttcagc tgcaagctca a 21
<210> 13
   <211> 21
   <212> DNA
   <213> artificial
<220>
   <223> reverse primer genotype A
<400> 13
   ccacaacacc ttgggtaatg c 21
<210> 14
   <211> 26
   <212> DNA
   <213> artificial
<220>
   <223> forward primer genotype B
<400> 14
   aatagggttt tcagctacca actcaa 26
<210> 15
   <211> 21
   <212> DNA
   <213> artificial
<220>
   <223> reverse primer genotype B
<400> 15
   ccacaacacc ttgggtaatg c 21
<210> 16
   <211> 21
   <212> DNA
   <213> artificial
<220>
   <223> forward primer genotype C
<400> 16
   gcatcgctca acctaaattg g 21
<210> 17
   <211> 21
   <212> DNA
   <213> artificial
<220>
   <223> reverse primer genotype C
<400> 17
   attgtggctt cccctaaaag g 21
<210> 18
   <211> 23
   <212> DNA
   <213> artificial
<220>
   <223> forward primer genotype D
<400> 18
   aaccacttgg aacccaacac ttt 23
<210> 19
   <211> 28
   <212> DNA
   <213> artificial
<220>
   <223> reverse primer genotype D
<400> 19
   cgaagcaagt tgtaagaagt cagagtaa 28
<210> 20
   <211> 22
   <212> DNA
   <213> artificial
<220>
   <223> forward primer genotype E
<400> 20
   ccaagccttc taggatcaag ga 22
<210> 21
   <211> 22
   <212> DNA
   <213> artificial
<220>
   <223> reverse primer genotype E
<400> 21
   cgaagcaatt tgcaagacat ca 22
<210> 22
   <211> 25
   <212> DNA
   <213> artificial
<220>
   <223> forward primer genotype F
<400> 22
   gcaacttttg atgctgactc tatcc 25
<210> 23
   <211> 24
   <212> DNA
   <213> artificial
<220>
   <223> reverse primer genotype F
<400> 23
   gttccatgtg gtcaagttca aaac 24
<210> 24
   <211> 22
   <212> DNA
   <213> artificial
<220>
   <223> Probe for Cp. psittaci genotype EB
<400> 24
   ccaagccttc taggatcaac ca 22
<210> 25
   <211> 20
   <212> DNA
   <213> artificial
<220>
   <223> forward primer genotype EB
<400> 25
   tgctttgccc aataatgctg 20
<210> 26
   <211> 26
   <212> DNA
   <213> artificial
<220>
   <223> reverse primer genotype EB
<400> 26
   aaggatgttc tatctgatgt cttgca 26
<210> 27
   <211> 101
   <212> DNA
   <213> Chlamydophila psittaci
<400> 27
   gttgggttaa tagggttttc agctgcaagc tcaatctcta ccgatcttcc aacgcaactt 60
   cctaacgtag gcattaccca aggtgttgtg gaattttata c 101
<210> 28
   <211> 101
   <212> DNA
   <213> Chlamydophila psittaci
<400> 28
   gttgggttaa tagggttttc agctaccaac tcaacctcta ccgatcttcc aatgcaactt 60
   cctaacgtag gcattaccca aggtgttgtg gaattttata c 101
<210> 29
   <211> 89
   <212> DNA
   <213> Chlamydophila psittaci
<400> 29
   gttggtttga ttggtgttaa aggaagctcc ttaacaaatg accaacttcc caacgtagcc 60
   atcactcaag gcgttgttga gttttacac 89
<210> 30
   <211> 86
   <212> DNA
   <213> Chlamydophila psittaci
<400> 30
   gttggtttga ttggtcttaa aggaactgat ttcaataatc aacttccaaa cgtagccatc 60
   acccaaggcg ttgttgagtt ttacac 86
<210> 31
   <211> 101
   <212> DNA
   <213> Chlamydophila psittaci
<400> 31
   gttgggttaa tagggttttc agctaccagc tcaacctcta ccgagcttcc aatgcaactt 60
   cctaacgtag gcattaccca aggtgttgtg gaattttata c 101
<210> 32
   <211> 89
   <212> DNA
   <213> Chlamydophila psittaci
<400> 32
   gttggtttga ttggtgttaa aggaacctcc gtagcagctg atcaacttcc aaacgtaggc 60
   atcactcaag gtattgttga gttttacac 89
<210> 33
   <211> 101
   <212> DNA
   <213> Chlamydophila psittaci
<400> 33
   atactatccg cattgctcaa cctaaattaa aatcggagat tcttaacatt actacatgga 60
   acccaagcct tataggatca accactgctt tgcccaataa t 101
<210> 34
   <211> 101
   <212> DNA
   <213> Chlamydophila psittaci
<400> 34
   atactatccg cattgctcaa cctaaattaa aatcggagat tcttaacatt actacatgga 60
   acccaagcct tctaggatca accactgctt tgcccaataa t 101
<210> 35
   <211> 95
   <212> DNA
   <213> Chlamydophila psittaci
<400> 35
   acactatccg catcgctcaa cctaaattgg cctctgctgt tatgaacttg accacatgga 60
   acccaaccct tttaggggaa gccacaatgc ttgat 95
<210> 36
   <211> 95
   <212> DNA
   <213> Chlamydophila psittaci
<400> 36
   acactatccg cattgctcag cctaaattag ccactgctgt tttagattta accacttgga 60
   acccaacact tttaggaaag gccacaactg tcgac 95
<210> 37
   <211> 101
   <212> DNA
   <213> Chlamydophila psittaci
<400> 37
   atactatccg cattgctcaa cctaaattaa aatcggagat tcttaacatt actacatgga 60
   acccaagcct tctaggatca accactactt tgcccaataa t 101
<210> 38
   <211> 95
   <212> DNA
   <213> Chlamydophila psittaci
<400> 38
   actctatccg catcgctcaa cctaaattag ccgctgctgt tttgaacttg accacatgga 60
   acccaactct tttaggggaa gctacagctt tagat 95
<210> 39
   <211> 101
   <212> DNA
   <213> Chlamydophila psittaci
<400> 39
   acattactac atggaaccca agccttatag gatcaaccac tgctttgccc aataatagtg 60
   gtaaggatgt tctatctgat gtcttgcaaa ttgcttcgat t 101
<210> 40
   <211> 101
   <212> DNA
   <213> Chlamydophila psittaci
<400> 40
   acattactac atggaaccca agccttctag gatcaaccac tgctttgccc aataatagtg 60
   gtaaggatgt tctatctgat gtcttgcaaa ttgcttcgat t 101
<210> 41
   <211> 92
   <212> DNA
   <213> Chlamydophila psittaci
<400> 41
   acttgaccac atggaaccca acccttttag gggaagccac aatgcttgat acttccaata      60
   aattcagtga cttcttacaa atcgcttcga tt 92
<210> 42
   <211> 92
   <212> DNA
   <213> Chlamydophila psittaci
<400> 42
   atttaaccac ttggaaccca acacttttag gaaaggccac aactgtcgac ggtaccaata 60
   cttactctga cttcttacaa cttgcttcga tt 92
<210> 43
   <211> 100
   <212> DNA
   <213> Chlamydophila psittaci
<400> 43
   acattactac atggaaccca agccttctag gatcaaccac tactttgccc aataatggtg 60
   gtaaggatgt tctatctgat gtcttgcaaa ttgcttcgat 100
<210> 44
   <211> 97
   <212> DNA
   <213> Chlamydophila psittaci
<400> 44
   acttgaccac atggaaccca actcttttag gggaagctac agctttagat gctagcaaca 60
   aattctgcga cttcttacaa atcgcttcga ttcagat 97
<210> 45
   <211> 101
   <212> DNA
   <213> Chlamydophila psittaci
<400> 45
   taaactggtc aagagcaact tttgatgctg atactatccg cattgctcaa cctaaattaa 60
   aatcggagat tcttaacatt actacatgga acccaagcct t 101
<210> 46
   <211> 101
   <212> DNA
   <213> Chlamydophila psittaci
<400> 46
   taaactggtc aagagcaact tttgatgctg atactatccg cattgctcaa cctaaattaa 60
   aatcggagat tcttaacatt actacatgga acccaagcct t 101
<210> 47
   <211> 101
   <212> DNA
   <213> Chlamydophila psittaci
<400> 47
   taaactggtc acgagcaact tttgatgccg acactatccg catcgctcaa cctaaattgg 60
   cctctgctgt tatgaacttg accacatgga acccaaccct t 101
<210> 48
   <211> 101
   <212> DNA
   <213> Chlamydophila psittaci
<400> 48
   taaactggtc aagagcaact tttgatgctg acactatccg cattgctcag cctaaattag 60
   ccactgctgt tttagattta accacttgga acccaacact t 101
<210> 49
   <211> 101
   <212> DNA
   <213> Chlamydophila psittaci
<400> 49
   taaactggtc aagagcaact tttgatgctg atactatccg cattgctcaa cctaaattaa 60
   aatcggagat tcttaacatt actacatgga acccaagcct t 101
<210> 50
   <211> 101
   <212> DNA
   <213> Chlamydophila psittaci
<400> 50
   taaactggtc aagagcaact tttgatgctg actctatccg catcgctcaa cctaaattag 60
   ccgctgctgt tttgaacttg accacatgga acccaactct t 101
<210> 51
   <211> 101
   <212> DNA
   <213> Chlamydophila psittaci
<400> 51
   acattactac atggaaccca agccttctag gatcaaccac tgctttgccc aataatgctg 60
   gtaaggatgt tctatctgat gtcttgcaaa ttgcttcgat t 101
<210> 52
   <211> 21
   <212> DNA
   <213> Chlamydophila psittaci
<400> 52
   atgaaaaaac tcttgaaatc g 21
<210> 53
   <211> 21
   <212> DNA
   <213> Chlamydophila psittaci
<400> 53
   acaagctttt ctagacttca t 21

## Description

### Field of the invention

The present invention relates to the qualitative and quantitative detection of genotypes of *Chlamydiaceae* as well as to the detection and diagnosis of bacterial infections in mammals, including humans and birds. The invention further relates to the detection of a novel strain of an infectious bacterium.

### Background of the invention

Bacteria in the family of the *Chlamydiaceae* are obligate intracellular parasites of eukaryotic cells. In animals, *Chlamydophilae are* capable of inducing a broad spectrum of symptoms like enteritis, urogenital infection, abortion, pneumonia, polyarthiritis, polyserositis, encephalitis and mastitis. *Chlamydophila (Cp.) psittaci* (formerly *Chlamydia psittaci*) causes respiratory diseases in birds and psittacosis or parrot-fever in man. Until now detection of *Cp. psittaci* in avian samples is routinely performed by direct visualisation of the organisms using cytological stainings, by isolation in cell culture or specific pathogen-free embryonated eggs, by detection of *Cp. psittaci* antigens or by serologic tests measuring antibodies. Cytological stainings have poor sensitivity and specificity and can only be used as a rapid preliminary investigation method. The main disadvantage of isolation is the need for viable bacteria. This means special requirements for collection and storage of samples, requirements that cannot always be fulfilled when collecting field samples. In addition, isolation is time-consuming and costly and can only be performed in laboratories with a specific biosafety level since *Cp. psittaci* is a zoonotic agent which spreads by aerosol. The current rapid antigen-detection methods are not recommended for demonstrating *Cp. psittaci* in individual birds because of shortcomings in either sensitivity or specificity. Serology is not particularly useful in diagnosing an active *Cp. psittaci* infection in birds because of the high prevalence of this infection in birds and the long-term (up to several months) persistence of anti-*Cp. psittaci* antibodies. In addition, antibody detection based on using whole organisms, LPS (LipoPolySaccharides) or outer membrane fractions can generate false positives due to the presence of antibodies cross reactive to the *Cp. psittaci* LPS or heat shock proteins. Importantly, current *Cp. psittaci* antibody detection tests cannot be used for demonstrating a *Cp. psittaci* infection in man, as humans can also become infected with other members of the Chlamydiaceae as *Chlamydia trachomatis, Chlamydophila pneumonieae* (formerly *Chlamydia pneumoniae*) and *Chlamydophila abortus* (formerly *psittaci* serotype 1) which can cause false-positive results. Diagnosis of infection with *Cp. psittaci* has been difficult and cumbersome. Until now, detection of *Cp. psittaci* in avian samples is done with serological tests, providing, as indicated above, only retrospective information.

*Cp. psittaci* has been classified into six avian serovars (A to F) using a panel of serovar-specific monoclonal antibodies against the Major Outer Membrane Protein (MOMP). The MOMP is encoded by the OmpA gene and OmpA restriction fragment length polymorphism (RFLP) analysis reveals six corresponding genotypes. Until now, genotype A, C and D are the most common genotypes associated with human psittacosis. SUDLER et al. ( "Molecular characterisation of chlamydial isolates from birds." VETERINARY MICROBIOLOGY,2004) discloses an in vitro method for the identification of DNA of genotypes A, B, F and G of C. psittaci, using 2 oligonucelotides specific for OmpA, followed by a restriction digest. The samples are from birds. While RFLP analysis of the *ompA* gene encoding the MOMP is allows specific detection of the *Cp. psittaci* genotypes, restriction patterns in RFLP are sometimes difficult to analyse, and *ompA* amplification cannot always be carried out directly on clinical samples. Moreover, this method requires the amplification of the entire 1200 bp *OmpA* gene which often fails when a limited amount of DNA is available. Indirect micro-immunofluorescence (IMIF) with monoclonal antibodies always requires culturing, and is therefore expensive and labour-intensive and is definitely less sensitive then genotyping by means of RFLP or whole *ompA* sequence analysis. Besides the interspecies diagnosis problems in the serological assays and the intraspecies difficulties when dealing with mixed infections in RFLP or serotyping, these tests all have the problem that they do not provide information about the actual number of infectious particles in the specimen, making it also difficult or impossible to follow up a treatment or to track down the origin of an infection. The present overview illustrates a need for a specific diagnostic test for determining the genotype of *Cp. psittaci* in birds and mammals including man. Such a test should be rapid and sensitive.

### Summary of the invention

In a first aspect, the invention relates to an *ex vivo* or *in vitro* method for the identification of the presence of one or more genotypes of *Cp. psittaci* in a sample. Thus the present invention provides a method for the determination of the presence of *Cp. psittaci* in a sample as well as a method to specifically identify amongst the different Cp. psittaci genotypes, which genotype is present in the sample, thus allowing the determination of the actively infecting agent, even when the samples is taken from an animal or human subject which has previously been infected with *Cp. psittaci.*

One specific embodiment of the invention relates to a method for detecting a novel genotype of *Cp. psittaci,* referred to as genotype EB. Further embodiments of the invention relate to methods for detecting and identifying the presence of the genotypes A, B, C, D, E, and F.

According to a further specific embodiment the ex vivo or in vitro method for the detection and/or identification of the presence of DNA of a genotype of *Cp. psittaci* in a sample comprises the steps of (a) incubating the sample with a first oligonucleotide which is capable of specifically hybridising to DNA of a genotype of *Cp. psittaci,* and, (b) determining the binding of the first oligonucleotide to DNA within the sample, which binding is indicative of the presence of DNA of a genotype of *Cp. psittaci* in that sample. According to specific embodiments of the invention the detection and/or identification is performed using a first nucleotide is comprising a sequence of at least 15 nucleotides of the OmpA gene of one of the *Cp. psittaci* genotypes, more specifically, comprising a sequence of at least 15 nucleotides within the region from about nucleotide 450 to about nucleotide 600 or from about nucleotide 900 to about 1100 of the OmpA sequence corresponding to GB accession AF269281 [SEQ ID. NO: 54], being essentially identical to a sequence of 15 nucleotides within the OmpA gene, more particularly within these regions of the OmpA gene. Most particular embodiments of the invention encompass methods whreein the first genotype-specific oligonucleotide is selected from the group consisting of sequence corresponding to SEQ ID NO: 1 for genotype A, sequence corresponding SEQ ID NO: 2, for genotype B, sequence corresponding SEQ ID NO: 3 for genotype C, sequence corresponding SEQ ID NO: 4, for genotype D, sequence corresponding SEQ ID NO: 5, for genotype E, sequence corresponding SEQ ID NO: 6, for genotype F and sequence corresponding SEQ ID NO: 25, for genotype EB or a sequence essentially identical thereto capable of hybridising specifically to the respective genotype.Such an oligonucleotide can be labeled e.g. with a chromophoric group at its 5' and with a quencher group at its 3' end. A particular embodiment of the invention relates to the identification of a particular genotype of *Cp. psittaci* in a sample. It is further envisaged that in alternative embodiments the probes of the present invention can be combined for the simultaneous detection of more than one genotype of *Cp. psittaci* in a sample.

A further aspect of the invention relates to an *ex vivo* or *in vitro* method for the identification of the presence of one or more (first) genotypes of *Cp. psittaci* in a sample as described above, wherein the specificity of the detection is further improved by the use of a second oligonucleotide which prevents non-specific hybridisation of the first oligonucleotide to the DNA of another genotype of *Cp. psittaci.* Thus, according to this embodiment of the invention, the sample is incubated with at least one second oligonucleotide in addition to the first oligonucleotide being capable of hybridising specifically to the DNA of a first *Cp. psittaci* genotype, whereby the second oligonucleotide is a competitor for the hybridisation of this first oligonucleotide to DNA of another genotype of *Cp. psittaci.* According to particular embodiments of this aspect of the invention the first and second oligonucleotide are selected from the group consisting of (a) a second oligonucleotide comprising the sequence of SEQ ID NO: 8, and a first oligonucleotide comprising the sequence of SEQ ID NO: 1, (b) a second oligonucleotide comprising the sequence of SEQ ID NO: 7, and a first oligonucleotide comprising the sequence of SEQ ID NO: 2; (c) a second oligonucleotide comprising the sequence of SEQ ID NO: 10, and a first oligonucleotide comprising the sequence of SEQ ID NO: 2, (d) a second oligonucleotide comprising the sequence of SEQ ID NO: 9, and a first oligonucleotide comprising the sequence of SEQ ID NO: 5, and (e) a second oligonucleotide comprising the sequence of SEQ ID NO: 11, and a first oligonucleotide comprising the sequence of SEQ ID NO: 5.

According to a particular embodiment of the method described in both the first and the second aspect of the present invention, the binding of the first oligonucleotide is determined by PCR amplification with a forward and a reverse primer. More particularly the forward and reverse primer are located about 1 to 100 bp 3' and 5' from the first oligonucleotide. Specific embodiments of the primers for use in detection of the first oligonucleotide in the context of the present invention are selected from group consisting of (a) primers comprising the sequence of SEQ ID NO: 12 and SEQ ID NO: 13, when the first oligonucleotide comprises the sequence of SEQ ID NO: 1; (b) primers comprising the sequence of SEQ ID NO: 14 and SEQ ID NO: 15 when the first oligonucleotide comprises the sequence of SEQ ID NO: 2; (c) primers comprising the sequence of SEQ ID NO: 16 and SEQ ID NO: 17 when the first oligonucleotide comprises the sequence of SEQ ID NO: 3; (d) primers comprising the sequence of SEQ ID NO: 18 and SEQ ID NO: 19 when the first oligonucleotide comprises the sequence of SEQ ID NO: 4; (e) primers comprising the sequence of SEQ ID NO: 20 and SEQ ID NO: 21 when the first oligonucleotide comprises the sequence of SEQ ID NO: 5; (f) primers comprising the sequence of SEQ ID NO: 22 and SEQ ID NO: 23 when the first oligonucleotide comprises the sequence of SEQ ID NO: 6; (g) primers comprising the sequence of SEQ ID NO: 25 and SEQ ID NO: 26 when the first oligonucleotide comprises the sequence of SEQ ID NO: 24; or primers which have a sequence essentially identical to the above primers for PCR amplification. have a sequence essentially identical to the above primers for PCR amplification.

Specific embodiments of the method according to both the first and the second aspect of the present invention are methods used for the detection and/or identification of a *Cp. psittaci* genotype in birds, most particularly for the detection in birds which are in a stage of development in which the maternal immunity disappears. One particular embodiment of the invention is a method for detecting and/or identifying an infection with *Cp. psittaci* in a duck of about 6 weeks after hatching.

Specific applications of the described embodiments of the method according to both the first and the second aspect of the present invention are the detection and/or identification of a *Cp. psittaci* infection in a sample in order to determine the efficacy of a treatment against a a *Cp. psittaci* infection. Thus the present invention further relate to methods for determining the efficacy of treatment of a a *Cp. psittaci* infection comprising the method steps described above.

Yet another aspect of the present invention relate to diagnostic kits for the detection and/or identification of a *Cp. psittaci* genotype comprising one or more oligonucleotides capable of hybridizing specifically to a sequence within the DNA of a genotype of *Cp. psittaci.* Particular embodiments of the diagnostic kit of the invention relate to kits wherein the one or more oligonucleotides are capable of hybridizing specifically to a sequence within the ompA gene of *Cp. psittaci.* Further specific embodiments relate to kits wherein the one or more oligonucleotides are capable of hybridizing specifically to a sequence within the region from about nucleotide 450 to about nucleotide 600 or from about nucleotide 900 to about 1100 of the OmpA gene sequence corresponding to GB accession AF269281 [SEQ ID. NO: 541]. Particular examples of the diagnostic kit of the invention relate to diagnostic kits for the identification of one or more of the genotypes selected from the group consisting of A, B, C, D, E, F and/or EB, whereby the EB genotype is a novel genotype of *Cp. psittaci* identified herein. comprising one or more of the oligonucleotides selected from the group consisting of:
- genotype-specific oligonucleotides: SEQ ID NO: 1, SEQ ID NO: 2, SEQ ID NO: 3, SEQ ID NO: 4, SEQ ID NO: 5, SEQ ID NO: 6 and 24; and
- genotype-specific primers: SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 25 and SEQ ID NO: 26

It will however be understood by the skilled person that genotype-specific oligonucleotides and genotype-specific primers essentially identical to the oligonucleotides and primers described therein can equally be applied in the context of the present invention. Further particular embodiments of the diagnostic kits of the present invention relate to kits comprising two oligonucleotides selected from the genotype-specific oligonucleotides SEQ ID NO: 1, SEQ ID NO: 2, SEQ ID NO: 3, SEQ ID NO: 4, SEQ ID NO: 5, SEQ ID NO: 6 and 24.

Yet a further aspect of the present invention relates to a novel strain of a *Cp. psittaci* bacterium, designated as *Cp. psittaci* genotype EB which is **characterized in that** it comprises the OmpA sequence depicted in SEQ ID NO: 51.

Yet another aspect of the present invention relates to a method of generating oligonucleotide sequences useful for the discrimination between at least two genotypes of *Cp. psittaci.* A particular embodiment of this aspect of the invention relates to a method comprising the steps of a) providing a multiple alignment of a part of the genomic sequence of at least two *Cp. psittaci* genotypes, b) identifying regions which contain sequence differences within that part of the genomic sequence, c) synthesizing one or more oligonucleotides comprising a sequence wherein the above-identified sequence differences occur. Most particularly such a genomic sequence sequence differences occur. Most particularly such a genomic sequence encodes a protein which causes pathogenicity, such as the OmpA protein. A particular embodiment of this aspect of the invention relates to a method whereby the part of the genomic sequence which is aligned to identify sequence differences comprises the sequence from about nucleotide 450 to about nucleotide 600 or from about nucleotide 900 to about nucleotide 1100 of the I OmpA sequence corresponding to GB accession AF269281 [SEQ ID. NO: 54].. Particular embodiments of this aspect of the invention relate to methods for generating oligonucleotide sequences useful for the discrimination between the genotype EB and another genotype of *Cp. psittaci.*

In yet a further aspect, the present invention provides oligonucleotides useful in the detection and/or identification of a *Cp. psittaci* genotype, most particularly the oligonucleotides selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, and SEQ ID NO: 26. As detailed above, the present invention demonstrates how these oligonucleotides can be employed in methods which allow the detection and/or specific identification of a *Cp. psittaci* genotype

The methods and kits of the present invention, provide several advantages over the current detection and/or identification methods, such as easy sample collection methods, simple transport and storage requirements of the bacterial sample, rapid results, the possibility for automatisation, and a high sensitivity and specificity.

The present invention allows the genotype-specific detection of *Cp. psittaci* which is based on the identification of the presence of DNA of the bacterium. It allows the detection of the presence or absence of *Cp. psittaci* bacteria in a against bacteria of a previous infection. Thus, contrary to serotypic detection methods, the methods and kits of the present invention allow the detection and/or identification of an active infection.

Moreover, the methods and kits of the present invention allow the species- and genotype-specific detection of *Cp. psittaci* in a sample, e.g. a sample from a human, which contains at the same time one more bacterial infections caused by one or more organisms selected from the group consisting of *Chlamydia trachomatis, Chlamydophila pneumonieae,* and *Chlamydophila abortus.*

The present invention further makes it possible to determine or to confirm and follow-up the relationship between the occurrence of a certain genotype and the pathogenicity thereof.

### DETAILED DESCRIPTION

### Definitions

**"Genotype"** as used in the present invention refers to the actual genetic composition of an organism as distinguished from its physical appearance (its phenotype). Thus while bacteria can have certain morphological properties which allow the determination of the organism up to the level of the genus, more subtle differences may occur which can only be attributed by sequence comparison of the whole genome or parts of the genome. Bacteria belonging to the same genus, in this invention *Cp. psittaci,* can have differences in certain regions of the genome (in a preferred embodiment the OmpA gene) and will accordingly be classified in different genotypes.
The bacterium ***"Chlamydophila* psittaci"** (abbreviated as *Cp. psittaci)* belongs to the class of chlamidiae and is described in Skerman, V.B.D., McGowan, V., and Sneath, P.H.A. (editors). "Approved lists of bacterial names." Int. J. Syst. Bacteriol. (1980) 30, 225-420. Synonyms which have been used are for this Bacteriol. (1980) 30, 225-420. Synonyms which have been used are for this bacterium are *Chlamydia psittaci, Chlamydozoon psittaci, Rickettsiaformis psittacosis, Ehrlichia psittaci* and *Rickettsia psittaci.* In animals, *Chlamydiaceae are* capable of inducing a broad spectrum of symptoms like enteritis, urogenital infection, abortion, pneumonia, polyarthiritis, polyserositis, encephalitis and mastitis. Several genotypes are known, designated A to F. Of these genotypes, A, C and D have most often been associated with human psittacosis. However the occurrence of psittacosis is underestimated, as routine genotyping tools are not available.

**"sample"** as used in the present iapplication refers to either a solid or liquid substance. In the context of the present invention, the sample is preferably a body sample, i.e. a sample obtained from the animal or human body e.g. a part of the body, a body fluid or any excretion or waste product. According to the present invention the sample will contain sample DNA, i.e. DNA originating from the body from which the sample is obtained.

Samples include but without being limited thereto, blood, any cellular part of the body, skin, sputum, mouth, pharyngeal, conjunctival nose or vaginal swabs, urine, faecal samples, breath samples comprising aerosols of bacteria or bacteria particles, any type of tissue samples and biopts, such as lung, airsac, spleen or liver or other organs. Equally the methods of the present invention can be performed on bacterially infected cell cultures. The method can be performed on a sample of living bacteria but also on a sample comprising dead bacteria as long as DNA of the gene fragment to be amplified with the present method is available. Due to its sensitivity the sample can comprise less than 10.000, less than 1000, less than 100 or even about 10 or less than 10 *Cp. psittaci* bacteria or copies of the DNA to be amplified according to the method of the present invention.

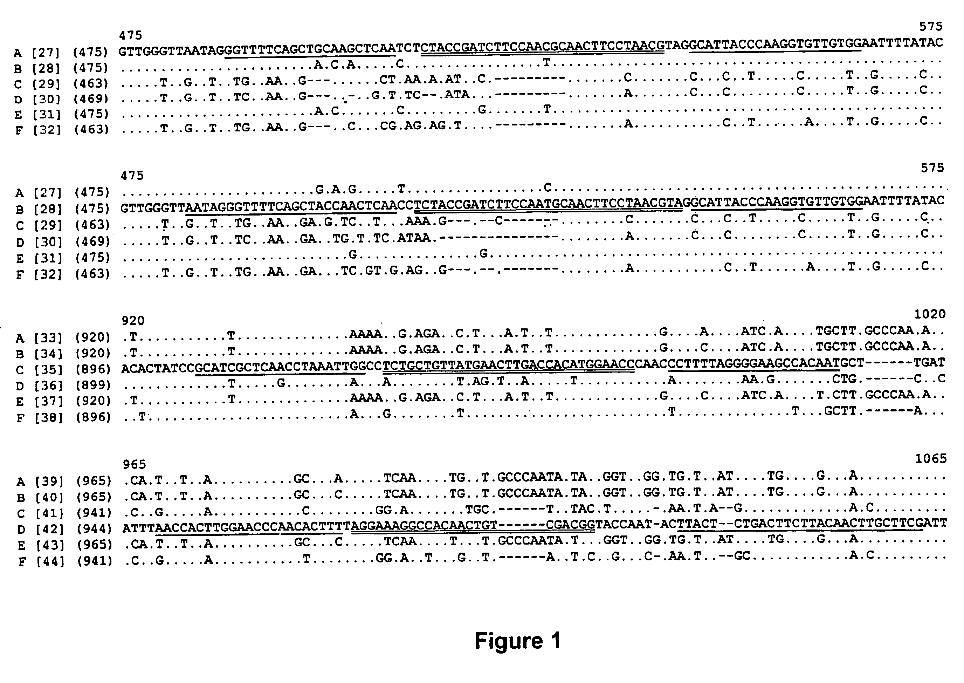

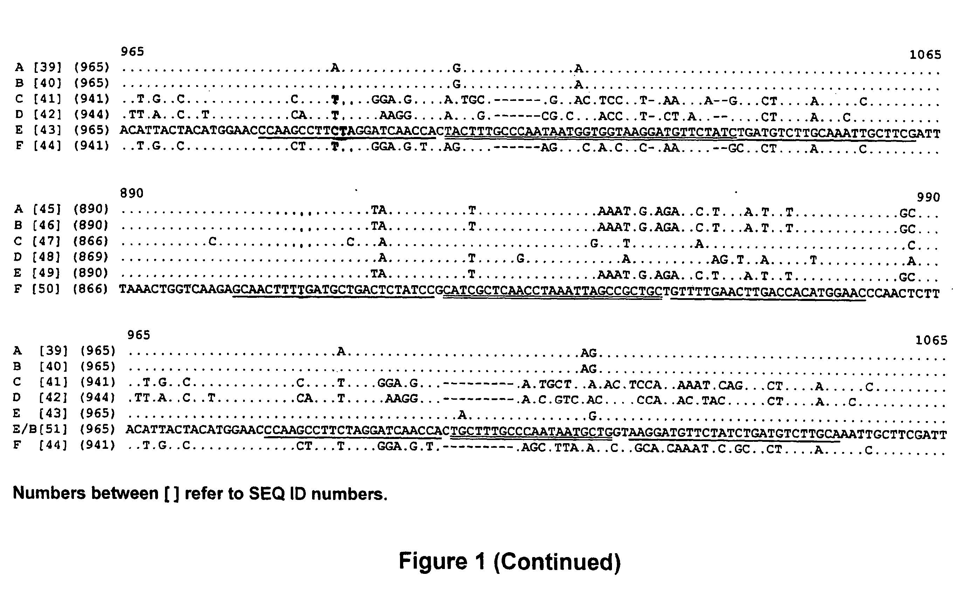

**"OmpA"** in the present invention refers to the Outer Membrane Protein A of *Cp. psittaci.* As an illustration, Genbank accession AF269281 [SEQ ID. NO: 54]. discloses the DNA and protein sequence of a strain of *Chlamydophila psittaci.* Partial sequences of *Cp. psittaci* OmpA from differing strains are presented in figure 1. under stringent conditions. Nucleic acid hybridisation will be affected by such conditions as salt concentration, temperature, or organic solvents, in addition to the base composition, length of the complementary strands, and the number of nucleotide base mismatches between the hybridising nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000 mM, typically less than 500 mM, and preferably less than 200 mM. A oligonucleotide capable of hybridising specifically to the DNA of a particular genotype of *Cp. psittaci* thus refers to a genotype capable of hybridising thereto under stringent conditions.

"Essentially identical" used herein in the context of a sequence which is essentially identical to a specific sequence of a first (or competitor) oligonucleotide provided herein refers to a sequence which differs in one to three nucleotides from the specific sequence provided. The nucleotides differing are nucleotides selected by the skilled person in such a way that they do not affect the specificity of the oligonucleotide towards its genotype DNA. Most particularly the nucleotides selected are nucleotides which are identical within the DNA sequence of the different genotypes of *Cp. psittaci.* In the context of the sequences of PCR primers provided, 'essentially identical' refers to a sequence which differs from the provided sequence at maximally 5 nucleotides without affecting its ability to function as a PCR primer for the respective first oligonucleotide.

The present invention relates to the specific detection and/or identification of a *Cp. psittaci* genotype in a sample. According to particular embodiments of the present invention the sample originates from a human or from a non-human mammal, such as cattle, pigs, cats, dogs, birds (such as poultry exemplified by ducks, chicken, ostriches, turkeys, racing and urban pigeons, and pet birds (e.g. parrots)). The present invention provides a genotype-specific genetic assay for diagnosis and treatment-follow-up of *Cp. psittaci* infections from respiratory samples of animals, such as are particularly well-described in birds (ornithosis) and humans (psittacosis).

In one embodiment, the sample is a sample of a bird that is in a stage of development when the maternal immunity of the bird disappears and infection with *Cp. psittaci* is likely. In general, maternal antibody titers against an infection decline and are almost absent by 3 to 4 weeks of age. Turkeys normally experience two *Cp. psittaci* infection waves, one at 3 to 4 weeks and the second at 8 to 10 weeks of ague. Accordingly the method of the present invention is advantageously performed on turkeys at these time points. Depending from animal species to species this time point on which the maternal immunity disappears varies. When the animal is a duck, the method is advantageously performed about 6 weeks after hatching of the egg. Further applications of the methods and kits of the present invention relate to the detection and/or identification of a *Cp. psittaci* genotype in a sample of an animal taken during or after the treatment against a *Cp. psittaci* infection or, in the case of e.g. poultry after the release from quarantine. The method of the present invention can be used in general to monitor the infection status of a poultry flock during production and for diminishing the risk of psittacosis in poultry workers. The method can also be used by public health officers to monitor the occurrence of the infection in risk groups as veterinarians and poultry workers. The method can be performed to evaluate the efficacy of treatment against a *Cp. psittaci* infection in both birds and humans. The method can also be used as a diagnostic control before releasing birds from quarantine or to monitor obligatory treatment during quarantine. The method can also be used to trace possible infection sources in case of human psittacosis outbreaks. The method can be used as taxonomic tool as it allows the detection of new genotypes. The method can also be used as a epidemiological tool for evaluating the relationship between the occurrence of a given genotype in birds and the risk of transmission to man as well as the relation between the occurrence of a genotype and the virulence thereof in both birds and mammals (especially humans).

Particular embodiments of the method of the invention are methods which comprise the steps of incubating a sample suspected of infection with *Cp. psittaci* with a first oligonucleotide, the first oligonucleotide being complementary to the DNA sequence of a genotype of *Cp. psittaci* allowing the hybridisation of a first oligonucleotide to DNA of *Cp. psittaci* present in a sample, and determining the binding of the first oligonucleotide within the sample. This last step ensures the identification of one or more genotypes of *Cp. psittaci.*

Particular embodiments of the methods of the present invention involve the use of different types of oligonucleotides. The 'genotype-specific' oligonucleotides also referred to as 'first oligonucleotides' used in the methods and kits of the present invention are oligonucleotides complementary to a DNA sequence of a *Cp. psittaci* genotype which is specific for this *Cp. psittaci* genotype and which is capable of hybridising specifically this specific sequence. In one embodiment of the invention capable of specifically hybridising refers to the ability of the oligonucleotide to hybridise specifically under hybridisation conditions which are commonly used during the elongation step of a PCR reaction.

The genotype-specific (first) oligonucleotides used in the context of the present invention can vary in length, between about 12 up to 30 or even 40 nucleotides, the proper length for an experiment being dependent on the technique used, the GC content of the probe used and the chance of non-specific binding of a probe to another target sequence. Specific embodiments of the invention, such as illustrated in the examples relate to probes of about 30 to 40 nucleotides. Differences can be envisaged wherein the probes are shorter or longer at their 3' and/or 5' end or are located more upstream or and/or downstream with respect to their target sequence (5, 10 15, 20 or more nucleotides). Particular embodiments of the first oligonucleotides suitable for use in the context of the present invention comprise or have the sequences in table 2 with SEQ ID NO: 1 (for genotype A), SEQ ID NO: 2 (for genotype B) , SEQ ID NO: 3 (for genotype C), SEQ ID NO: 4 (for genotype D), SEQ ID NO: 5 (for genotype E), SEQ ID NO: 6 (for genotype F) and SEQ ID NO: 24 (for genotype EB). It will however be understood that sequences essentially identical to the sequences described herein can be designed for use in the context of the present invention.

In a particular embodiment the first oligonucleotide is labeled with a chromophoric group at its 5' and with a quencher group at its 3' end, in order to be suitable for use in a quantitative PCR method (e.g. so called "taqman"). Suitable labels include but are not limited to e.g. the fluorescent indicator molecules selected from the group consisting of fluorescein, rhodamine, texas red, FAM, JOE, *TAMRA,* ROX, HEX, TET, Cy3, Cy3.5, Cy5, Cy5.5, IRD40, IRD41 and BODIPY.

The binding of an oligonucleotide to DNA present in a sample can be determined via a variety of techniques such as southern or northern hybridisation and chromatography under denaturing conditions. In one embodiment of the invention, the binding of an oligonucleotide can be determined by evaluating the binding of an identical non-labeled oligonucleotide for the same binding site. e.g. replacement of a chromogenic probe by a non chromogenic probe or vice versa. In a particular embodiment, the replacement of the non-chromogenic probe occurs during a PCR reaction wherein a quencher group is removed from a probe by DNA polymerase.

According to a specific embodiment, the methods of the invention are quantitative real-time PCR assays. It is demonstrated herein that the assays of the invention meet the criteria proposed for a validated assay, as both new real-time PCR assays were compared with other assays such as *ompA* sequencing, *ompA* RFLP and MOMP serotyping. Real-time PCR technology offers a new diagnostic approach which allows amplicon quantification in one step via specific hybridisation, without the need to open tubes, minimising the risk of cross-contamination for further experiments in this way.

The present invention further presents a method of generating genotype specific antibodies, which are derived from peptides having a sequence located within one of the sequences depicted in figure 1. Oligopeptides having a unique sequence for a certain genotype are used for the generation of antibodies.

According to a specific aspect of the methods and kits of the present invention second or competitor probes are used in combination with the first genotype-specific oligonucleotides of the invention. The second or competitor probes of the present invention are genotype-specific probes directed against another *Cp. psittaci* genotype DNA which genotype is different from the one which is envisaged to be detected and prevents non-specific binding of the first oligonucleotide according to the present invention to said other *Cp. psittaci* genotype. Thus, according to this aspect, the method comprises incubating the sample in addition to the first oligonucleotide with a second oligonucleotide (so called competitor) and determining the binding of the first oligonucleotide to DNA within the sample. Depending from the first oligonucleotide used, different competitors can be suitable for ensuring the increased specificity of the detection. According to one embodiment the first oligonucleotide corresponds to one of the sequences selected from SEQ ID NO: 1 to 6 or 24 described herein and the competitor oligonucleotide corresponds to a sequence comprising the nucleotide sequence in the OmpA gene which can be aligned with another one of the sequences of SEQ ID NO: 1 to 6 or 24. Most particularly, for the detection of genotype A, the first oligonucleotide is corresponds to SEQ ID NO: 1 and the competitor oligonucleotide is a sequence corresponding to SEQ ID NO: 1 within the sequence of the OmpA gene of the genotype B, C, D, E , F or EB (after alignment of the OmpA sequence of genotype A to that of genotype B, C, D, E, F or EB). The following embodiments represent examples of suitable combinations of competitors and probes:
- the second oligonucleotide comprises the sequence of SEQ ID NO: 8, and the first oligonucleotide comprises the sequence of SEQ ID NO: 1;
- the second oligonucleotide comprises the sequence of SEQ ID NO: 7, and the first oligonucleotide comprises the sequence of SEQ ID NO: 2;
- the second oligonucleotide comprises the sequence of SEQ ID NO: 10, and wherein the first oligonucleotide comprises the sequence of SEQ ID NO: 2;
- the second oligonucleotide comprises the sequence of SEQ ID NO: 9, and the first oligonucleotide comprises the sequence of SEQ ID NO: 5;
- the second oligonucleotide comprises the sequence of SEQ ID NO: 11, and the first oligonucleotide comprises the sequence of SEQ ID NO: 5.

Again, it will however be understood that sequences essentially identical to the sequences described herein can be designed for use in the context of the present invention. Moreover, it will be understood that further competitor oligonucleotides can be designed by the skilled person to avoid non-specific hybridisation of a first oligonucleotide of the invention with a DNA sequence of a genotype other than the one against which it is directed.

As detailed above, according to a particular embodiment, the method of detection of the binding is PCR. In a preferred embodiment the binding of a first and/or binding of a second oligonucleotide is determined by PCR amplification with a forward and a reverse primer, wherein the forward and reverse primer are located about 1, 5, 10, 20, 50 to 100 bp 3' and 5' from the first or second oligonucleotide. The PCR may be real-time PCR. Multiplexing can be used to reduce time.

The following embodiments represent examples of suitable pairs of forward and reverse primers for respective first oligonucleotides:
- primers comprising or containing the sequence of SEQ ID NO: 12 and SEQ ID NO: 13 when the first oligonucleotide comprises or contains the sequence of SEQ ID NO: 1.
- primers comprising or containing the sequence of SEQ ID NO: 14 and SEQ ID NO: 15 when the first oligonucleotide comprises or contains the sequence of SEQ ID NO: 2.
- primers comprising or containing the sequence of SEQ ID NO: 16 and SEQ ID NO: 17 when the first oligonucleotide comprises or contains the sequence of SEQ ID NO: 3.
- primers comprising or containing the sequence of SEQ ID NO: 18 and SEQ ID NO: 19 when the first oligonucleotide comprises or contains the sequence of SEQ ID NO: 4.
- primers comprising or containing the sequence of SEQ ID NO: 20 and SEQ ID NO: 21 when the first oligonucleotide comprises or contains the sequence of SEQ ID NO: 5.
- primers comprising or containing the sequence of SEQ ID NO: 22 and SEQ ID NO: 23 when the first oligonucleotide comprises or contains the sequence of SEQ ID NO: 6.
- primers comprising or containing the sequence of: SEQ ID NO: 25 and SEQ ID NO: 26 when the first oligonucleotide comprises or contains the sequence of SEQ ID NO: 24.

In another aspect the invention relates to isolated oligonucleotides comprising a or containing a sequence selected from the group of consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ 10 NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 SEQ ID NO: 24, SEQ ID NO: 25 and SEQ ID NO: 26, and sequences which are essentially identical thereto.

In another aspect the invention relates to a diagnostic kit comprising one or more oligonucleotides capable of specifically hybridizing to a DNA sequence of a genotype of *Cp. psittaci.* According to a particular embodiment the diagnostic kits comprise one or more of the first genotype-specific oligonucleotides capable of hybridising specifically to a genotype of the *Cp. psittaci,* most specifically one of the oligonucleotides selected from the group consisting of SEQ ID NO: 1 to 6 and SEQ ID NO: 24. According to a further embodiment the kit can additionally comprise one or more competitor probes, more specifically, one or more of the competitor oligonucleotides selected from the group consisting of SEQ ID NO: 8 to 11. Additionally or alternatively the kits of the present invention can comprise two primers, more particularly primer pairs selected from the group consisting of SEQ ID NO: 12 and 13, SEQ ID NO: 14 and 15, SEQ ID NO: 16 and 17, SEQ ID NO: 18 and 19, SEQ ID NO: 20 and 21, SEQ ID NO: 22 and 23, SEQ ID NO: 25 and 26. The kit can be further supplemented with e.g. reference strains of *Cp. psittaci* bacteria, plasmids containing a complete or partial OmpA DNA sequence of reference genotypes, or antibodies against *Cp. psittaci.* Other components can be bacteria or DNA samples of bacteria closely related to *Cp. psittaci.*

Another aspect of the invention relates to a novel strain of a *Cp. psittaci* bacterium, designated as *Cp. psittaci* genotype EB. This novel strain is **characterized in that** its genome comprises the specific sequence of the OmpA gene depicted in SEQ ID NO: 51. As detailed herein, the identification of this novel strain allows a more specific identification of the genotypic strains of *Cp. psittaci* in a sample. The present invention further provides EB-genotype-specific sequence SEQ ID NO: 51 and the use of this sequence and (EB-specific) fragments thereof in different applications, such as, but not limited to the specific detection of *Cp. psittaci* EB genotype, the generation of antibodies against corresponding amino acids sequences, etc..

Another aspect of the invention relates to a method of generating oligonucleotide sequences useful for the discrimination between at least two genotypes in the detection of *Cp. psittaci,* the method comprising the steps of:
a) providing a (multiple) alignment of a part of the genomic sequence of the at least two *Cp. psittaci* genotypes, b) identifying regions which contain sequence differences within said part of the genomic sequence, and c) synthesizing one or more oligonucleotides comprising a sequence wherein said sequences differences occur. According to one embodiment the genomic sequence used comprises a sequence for a gene encoding a protein which causes pathogenicity. In another embodiment, one of the at least two genotypes of *Cp. psittaci* is of the genotype EB.

The present invention is further illustrated with the following Figures and Examples, not intended to limit the scope of the invention.

### Figure legends

- **Figure 1:**: alignment of parts of the OmpA sequence of different *Cp. psittaci* strains (genotypes) with probes (double underlined) and forward and reverse primers (underlined) in accordance with an embodiment of the present invention.
- **Figure 2:**: genotype-specific standard curves obtained with the GeneAmp 5700 apparatus.
- **Figure 3:**: quantitative PCR results of VS-study mixed infections

### Example 1. : General methodology

**Isolates and cell cultures.** *Cp. psittaci* genotype A to F plus E/B reference strains 90/1051, 41A12, GD, 7344/2, 3759/2, 7778B15 and WS/RT/E30 (Table 1), were grown in Buffalo Green Monkey (BGM) cells. Infected monolayers were disrupted by freezing and thawing followed by ultrasonic treatment for 1 minute in a tabletop sonicator (Bransonic 12, BIOMEDevice, San Pablo, CA, USA). The cell culture harvest was centrifuged for 10 min (1,000 × g, 4° C) to remove cellular fragments and subsequently concentrated by ultracentrifugation for 1 hour (45,000 × g, 4°C). Bacterial pellets were resuspended in Sucrose Phosphate Glutamate buffer (SPG) (218 mM sucrose, 38 mM KH₂PO₄, 7 mM K₂HPO₄, 5 mM L-glutamic acid) at a volume of 1 to 100 of the original culture volume and stored at -80 °C until use.

**DNA extraction**. Genomic DNA was prepared as follows. 200 µl cell culture harvest was centrifuged for 30 min at RT (16,000 × g). The supernatant was discarded and the pellet resuspended in 199 µl SET buffer pH 7,5 (0.05 M Tris, 0.01 M EDTA, 1 % SDS) supplemented with 1 µl Proteinase K (20 mg/ml, Promega, Madison, WI, USA). Samples were incubated at 37°C for 30 min and subsequently boiled for 10 min to inactivate the enzyme.

**Genotype-specific reference plasmid constructions.** The *ompA* gene of the genotype A to F plus E/B reference strains (Table 1) was amplified resulting in a fragment of 1,065 to 1,098 bp depending on the genotype. Primers were chosen from the highly conserved regions of the published *ompA* sequences of *C. trachomatis* and *Cp. psittaci.* Amplification of the *ompA* gene was accomplished using the genol [SEQ ID NO:52] and genoll [SEQ ID NO: 53] primers (Table 2) syntesized by Invitrogen. Thirty-five cycles of 1 min denaturation at 95 °C, 2 min annealing at 55 °C and 3 min extension at 72 °C were completed in a Perkin Elmer GeneAmp 9600 after an initial denaturation of 5 min at 95°C and followed by 5 min end annealing at 72 °C.

**Table 1: Cp. psittaci reference plasmids**

| Plasmid | Strain | Country (year) | Host | Genotype |
|---|---|---|---|---|
| 22A | 90/1051 | Belgium (1990) | *Amazona sp.* | A |
| 29B | 41A12 | Belgium (2001) | *Meleagris gallopavo* | B |
| 45A | GD | Gemany (1960) | *Anas platyrhyncos* | C |
| 19A | 7344/2 | Italy (1997) | *Columba livia* ^{a} | D |
| 17A | 3759/2 | Italy (1999) | *Columba livia* ^{a} | E |
| 32B | 7778B15 | Belgium (2001) | *Meleagris gallopavo* | F |
| 35A | WS/RT/E30 | Germany (2001) | *Anas platyrhyncos* | EB |

| | | | | |
|---|---|---|---|---|
| ^{a} Isolated from an urban pigeon | | | | |

**Table 2: PCR primers, probes and competitors for genotype specific detection of Cp psittaci.**

| **Oligo** | **Sequence (5'-3')** | **Melting Point (°C)** | **SEQ ID. NO:** |
|---|---|---|---|
| Genotype A probe | (or other chromphoric and/or quencher group) | 69 | 1 |
| Genotype A forward | 5'-GGTTTTCAGCTGCAAGCTCAA-3' | 59 | 12 |
| Genotype A Reverse | 5'-CCACAACACCTTGGGTAATGC-3' | 59 | 13 |
| Genotype B probe | (or other chromphoric and/or quencher group) | 69 | 2 |
| Genotype B forward | 5'-AATAGGGTTTTCAGCTACCAACTCAA-3' | 59 | 14 |
| Genotype B reverse | 5'-CCACAACACCTTGGGTAATGC-3' | 59 | 15 |
| Genotype C probe | (or other chromphoric and/or quencher group) | 69 | 3 |
| Genotype C forward | 5'-GCATCGCTCAACCTAAATTGG-3' | 58 | 16 |
| Genotype C reverse | 5'-ATTGTGGCTTCCCCTAAAAGG-3' | 58 | 17 |
| Genotype D probe | (or other chromphoric and/or quencher group) | 68 | 4 |
| Genotype D forward | 5'-AACCACTTGGAACCCAACACTTT-3' | 60 | 18 |
| Genotype D reverse | | 60 | 19 |
| Genotype E probe | | 68 | 5 |
| Genotype E forward | 5'-CCAAGCCTTCTAGGATCAAGGA-3' | 59 | 20 |
| Genotype E reverse | 5'-CGAAGCAATTTGCAAGACATCA-3' | 60 | 21 |
| Genotype F probe | | 68 | 6 |
| Genotype F forward | 5'-GCAACTTTTGATGCTGACTCTATCC-3' | 59 | 22 |
| Genotype F Reverse | 5'-GTTCCATGTGGTCAAGTTCAAAAC-3' | 58 | 23 |
| Genotype EB Probe | 5'-CCAAGCCTTCTAGGATCAACCA-3' | | 24 |
| Genotype EB Forward | 5'-TGCTTTGCCCAATAATGCTG-3' | | 25 |
| Genotype EB Reverse | 5'-AAGGATGTTCTATCTGATGTCTTGCA-3' | | 26 |
| Genotype A competitor B CpPsGAcomB | | | 8 |
| Genotype B competitorA CpPsGBcomA | | | 7 |
| Genotype B | 5'-TCTACCGAGCTTCCAATGCAA- | | 10 |
| competitor E + E/B CpPsGBcom E + E/B | CTTCCTAACGTA-3' | | |
| genotype E competitor E/B CpPsGEcomE B | | 69 | 9 |
| Genotype E competitorAB CpPsGEcomA B | | | 11 |
| Genol | 5'-ATGAAAAAACTCTTGAAATCG-3' | 55 | 52 |
| Genoll | 5'-ACAAGCTTTTCTAGACTTCAT-3' | 55 | 53 |

**Quantitative *ompA* Genotype specific real-time PCR.** *Cp. psittaci* genotype specific PCR primers were selected from the variable segments of the *ompA* gene with primer express software (Applied biosystems) and synthesized by Invitrogen. The PCR products generated were between 78 and 85 bp depending on the genotype. Sequences of the primers and TaqMan probes (synthesized by Applied Biosystems) for the different genotypes are presented in Table 2. The genotype specific probes were 5' labelled with 6-carboxyfluorescein (FAM) as the reporter dye and with 6-carboxythetramethylrhodamine (TAMRA) at the 3' end as the quencher. Other dye - quencher combinations can be used as alternatives. A sequence alignment of parts of the OmpA gene and the probes being used are shown in figure 1. For the A, B and E genotypes, competitor oligo's were used to enhance the specificity of the probe. Forward and reverse primers and probes were tested in concentrations of 50, 150, 300 and 900 nM, with and without adding the competitor DNA (50 nM or 150 nM), supplemented with purified genomic DNA of the six genotype reference strains. Best results were achieved with forward and reverse primer concentrations of 300 nM, a probe concentration of 300 nM and where applicable, a competitor concentration of 50 nM. Cycling conditions were those suggested by the manufacturer and all default program settings were used. PCR was performed in ABI PRISM® optical tubes (Applied Biosystems), with the reaction mixtures consisting of 25 µl of the TaqMan universal Master mix including dUTP and uracyl N-glycosylase (AmpErase UNG; Applied Biosystems), in a total reaction volume of 50 µl. Amplification and detection of the PCR product was performed with an ABI GeneAmp 5700 sequence detection instrument (Applied Biosystems), using all default program settings. Cycling conditions were as follows: after 2 min 50 °C and 10 min at 95 °C, the samples were submitted for 40 cycles, each consisting of an initial denaturation step at 95 °C for 15 s followed by a step at 60 °C for annealing and extension for one minute. The PCR products were detected as an increase in fluorescence during the PCR extension phase when the probe was cleaved by the 5' exonuclease activity of the *Taq* DNA polymerase. Standard graphs of the Ct values obtained from serial dilutions of purified reference plasmids (10⁸ to 10¹) were constructed. Ct values for unknown clinical samples were plotted against the standard graphs for plasmids. Finally, the amount of the different *Cp. psittaci* genotypes present in the clinical samples (No) was. In addition, DNA from each clinical sample was tested in the presence of *Cp. psittaci* DNA (50 *ompA* copies for each genotype) to check for PCR inhibitors by comparing the amplification plots for the samples with and without this internal controls.

Identical or similar settings can be used in apparatus from other manufactures in order to reproduce the disclosure of the present invention.

**Positive controls and constructed test samples**. Mixtures of plasmids with OmpA of known concentration can be used as a model for mixed cultures of *Cp. psittaci* because *OmpA* occurs as single copy gene in the bacterium.

**Clinical samples.** *Ornithosis*/*psittacosis study.* In an experiment with five groups of SPF turkeys (5.07, 5.09, 5.10, 5.11 and 5.12), animals of each group were dying due to an unknown cause, having severe respiratory symptoms. Pharyngeal swabs from each group of animals were collected by serial passage through the five animals in each group, as well as from the veterinarian who took care of them to verify whether *Cp. psittaci* was the causative agent. A second swab of the veterinarian was taken two weeks later. Swabs were shaken in ml sucrose phosphate glutamate buffer (SPG, 218 mM sucrose, 38 mM KH₂PO₄, 7 mM K₂HPO₄, 5 mM L-glutamic acid). One-day-old HeLa monolayers were inoculated with the supernatant and examined with the Chlamydia Imagen kit (DakoCytomation) according to the manufacturers instructions. In parallel, 100 µl suspension was centrifuged (10 min2700 × g) and used for DNA extractions with the SET-method.

***Longitudinal study**.* A longitudinal study was performed on three turkey farms in order to examine the kinetics of avian pneumovirus (APV), *Omithobacterium rhinotracheale* (ORT)_{;} *M. gallisepticum, M. meleagridis* and *Cp. psittaci* infections from day one until slaughter. Pharyngeal swabs from week 3, 6, 8, 12 and 15 after hatching were used for DNA extraction with the SET-method to quantify the presence of *Cp. psittaci* and to compare this result with the antibody response of the animals during the infection as determined by ELISA ***VS-study.*** In a previous study performing whole ompA sequencing of several clones per isolate revealed the presence of 5 mixed-genotype infections on a total of 21 isolates. Genomic DNA extractions of these isolates were used to verify the presence of the genotypes found by sequencing with the genotype specific RT-PCR-reactions.

### Example 2. Genotype specific identification of Cp. psittaci

The present invention demonstrates for the fist time the use of real time PCR technology to detect seven different avian *Cp. psittaci* genotypes in human and animal samples and offers the possibility to discover new *Cp. psittaci* genotypes.

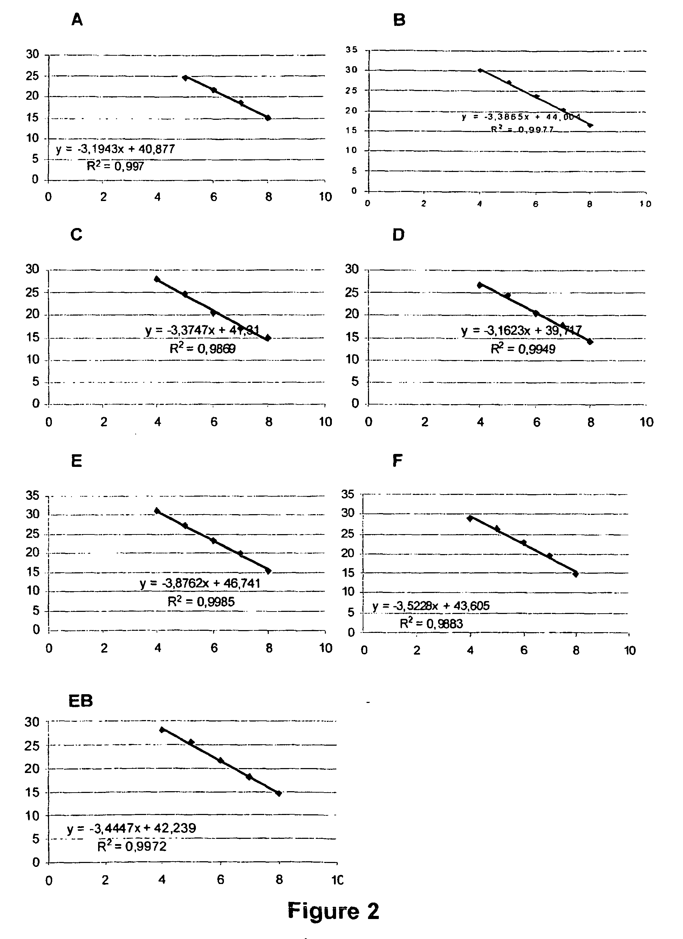

Using genotype specific reference plasmids, all seven PCR's (A to EB) are able to detect 10 copies of plasmid per µl. Standard curves could be made from 10⁸ to 10⁵ copies per µl with almost ideal slopes around -3,3 and correlation coefficients higher then 98,5% (Figure 2). The highest dilutions were not taken into account for the regression because the reproducibility was too low, they reached the threshold around the same cycle or only after cycle 40.

The competitors which have been used in the PCR methods of the present invention are oligonucleotides without a fluorescent signal that go in competition with probes that bind to the target sequence. In Fluorescence In Situ Hybridization (FISH) they are frequently used to enhance specific binding of the probes by blocking the possible probe sites on contaminating DNA. Competitors were until now never used in RT-PCR. This principle disclosed in this invention is applicable in any type of PCR reaction, wherein a probe is used which resides between the forward and reverse primer and wherein a further oligonucleotide is being used which competes with the probe for binding to the template DNA.

When investigating the primer and probe specificity of the reactions by preparing a mixture of 1/10 dilutions of genomic DNA extracts of the different genotypes plus undiluted, 1/100 and 1/1000 diluted material of the specific genotype, the results indicated that the C, D and F primers and probes did not render any significant reaction with the 1/10 dilution of the other genotype extracts, but the A, B, E and EB probes on the other hand did react with the other genomic material present. The development of genotype specific competitors allowed to differentiate all seven genotypes when added in a concentration of 50 nM. Competitor sequences are shown in Table 2.

### Example 3: Genotype determination

*Genotype A.* The *Chlamydophila psittaci* genotype A specific competitor for binding on genotype B (CpPsGAScomB) [SEQ ID NO:8] has to be added to the reaction mixture to prevent false positive results if genotype B is possibly present in the sample. When added, the competitor will bind the genotype B DNA, leaving the probe only the binding site on genotype A, if present. As the competitor sequence is complementary to the genotype B sequence, the affinity is higher for this genotype, while the probe off course preferentially binds genotype A.

*Genotype B.* In genotype B determination, an elevated temperature can enhance the probe specificity: aspecific reactions with genotypes E and EB disappear when the reaction is carried out at 63 °C in stead of 60 °C. Addition of the competitor for genotype A material CpPsGBScomA [SEQ ID NO:7] will prevent false positive reactions if genotype A material is present.

*Genotype E.* Addition of both CpPsGEScomA/B [SEQ ID NO:11] (competitor to prevent binding of probe E to genotype A and to genotype B) and CpPsGEScomEB [SEQ ID NO:9] (to prevent binding to EB) in equal concentrations of 50 nM prevent reaction with A and B efficiently, while the false positive signal EB comes several cycli later and the intensity is of it is reduced to 25 % of the specific E signal.

### Example 4: genotype specific detection of Cp. psittaci on clinical samples. Ornithosis/psittacosis study.

Samples 5.07, 5.09, 5.10, 5.11 and 5.12 from the turkeys as well as the two samples of the veterinarian (V1 and V2) were all positive in DIF three days post inoculation. When the genotype specific RT reactions were carried out directly on the sample resuspended in SPG, there was no reaction. Addition of the internal controls (50 copies/µl of the reference plasmids) proved that this was due to inhibition of the reaction. After SET-DNA extraction, reactions were done again and results showed that all turkeys were infected with the genotypes D, F and EB. On the same moment, the veterinarian already seemed infected with genotypes D and EB. The second sample of the veterinarian showed the genotypes D, F and EB to be present. Standard curves were made with 10⁷, 10⁵ and 10³ reference plasmids per µl on an ABI prism 7000 and Ct's of the samples were determined and plotted against the standard curves to determine the number of particles for each genotype. Results are shown in Table 3. These results show the zoonotic effect of *Cp. psittaci*: although there were no visible clinical symptoms, the veterinarian became infected with the same genotype strains as the turkeys. On the first timepoint genotype F was not yet detected, but sample V2 shows that genotype F had the chance to multiplicate in an incubation time of two weeks (Table 3).

**Table 3: Quantification analysis ornithosis/psittacosis study on samples of birds (5.07, 5.09, 5.10, 5.11, 5.12) and humans (V1/V2)**

| | | **5.07** | | | **5.09** | | | **5.10** | | |
|---|---|---|---|---|---|---|---|---|---|---|
| **genotype** | **3 point std curve** | **C_{T}** | **X- value** | **Copies / µl** | **C_{T}** | **X- value** | **copies/µl** | **C_{T}** | **X-value** | **copies/µl** |
| **D** | **Y= -2,74X + 37,62** | **34,37** | **1,18** | **15** | **31,43** | **2,26** | **182** | **33,1** | **1,65** | **45** |
| **F** | **Y= -3,20X + 40,89** | **35,18** | **1,78** | **61** | **29,55** | **3,54** | **3497** | **32,22** | **2,70** | **512** |
| **EB** | **Y = -2,7X + 37,37** | **31,3** | **2,25** | **177** | **29,57** | **2,89** | **772** | **31,28** | **2,25** | **180** |
| **Geno -type** | **3 point std curve** | **5.11 C_{T}** | **X-value** | **copies/** µ**l** | **5.12 C_{T}** | **X-value** | **copies/** µ**l** | **V1/V2 C_{T}** | **X-value** | **copies/** µ**l** |
| **D** | **Y = -2,74X +37,62** | **32,97** | **1,70** | **50** | **34,69** | **1,07** | **12** | **34,49 /35,99** | **1,14 /0,59** | **14 16** |
| **F** | **Y= -3,20X + 40,89** | **35,27** | **1,76** | **57** | **35,88** | **1,57** | **37** | **- /33,4 -** | **/2,34 -** | **/220** |
| **EB** | **Y = -2,7X + 37,37** | **31,85** | **2,04** | **110** | **31,34** | **2,23** | **171** | **31,99 /31,22** | **1,99 /2,28** | **98 /189** |

***Longitudinal study.*** DNA extracts from swabs after 3, 6, 8, 12 and 15 weeks after hatching were screened in the species specific PCR in a Perkin Elmer GeneAmp 9600 apparatus (Wellesley, MA, USA) without SybrGreen. All samples showed the characteristic 151 bp amplicon, already proving that the animals were infected with a *Cp. psittaci* genotype B strain and that two infections (week 6 and 12) were found on the farm. A genotype B standard curve with 10⁷, 10⁵ and 10³ reference plasmids per µl was made on an ABI prism 7000 and Ct's of the samples were determined and plotted against the standard curves to determine the number of particles. The genotype B specific real time PCR could prove that the high antibody responses were indeed correlated with a tenfold increase in *Cp. psittaci* genotype B (see week 6 and 12, in Table 4).

**Table 4: Quantification analysis of the longitudinal study**

| **Week** | **0** | **1** | **2** | **3** | **4** | **5** | **6** | **7** |
|---|---|---|---|---|---|---|---|---|
| Titer | 3072 | 768 | 768 | 1536 | 768 | / | 3072 | / |
| Copies/µl | /^{a} | / | 24 | / | / | / | 217 | 8 |

| **Week** | **8** | **9** | **10** | **11** | **12** | **13** | **14** | **15** |
|---|---|---|---|---|---|---|---|---|
| Titer | 1536 | / | 768 | / | 3072 | / | 768 | / |
| Copies/µl | 33 | / | / | / | 238 | / | / | 19 |

| | | | | | | | | |
|---|---|---|---|---|---|---|---|---|
| ^{a} / not available ; calculation was done with the genotype B standard curve y = - 2,92 x + 39,53 with y = Ct and x = log (copies / µl) | | | | | | | | |

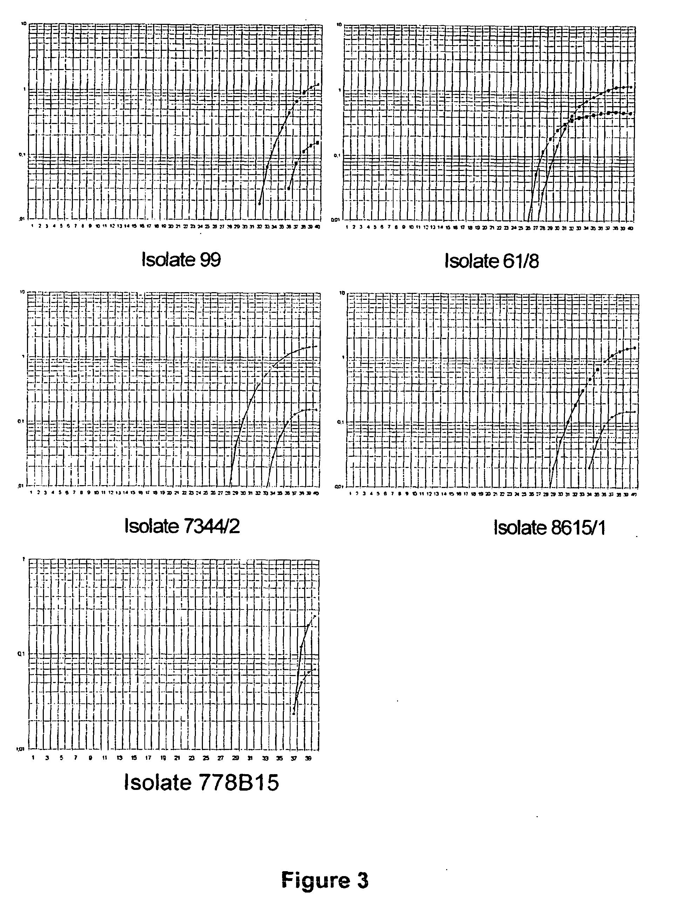

***VS-study.*** Isolates revealing mixed infections were submitted to the genotype specific real-time pcr reactions to confirm the presence of the different genotypes indicated by the whole *ompA* sequencing. Table 5 shows that all mixed infections could be detected easily and moreover, quantified using the Ct values determined on the graphs presented in Figure 3 and the standard curves of Figure 2. The genotype that is less abundant remains undetected in four of the five cases.

In addition to the specificity of the quantitative PCR method to discriminate genotypes, the specificity was also tested on DNA extracted from other bacterial species commonly found in the avian and human respiratory tract as well as on DNA extracted from avian (HD11) and (Hela) cells. No amplified DNA prodcuts were detected.

**Table 5: Quantification analysis VS-study**

| **Isolate** | ***OmpA* sequencing** | ***OmpA* RFLP** | **MOMP serotyping** | **Ct** | **X** | **N₀^{a}** |
|---|---|---|---|---|---|---|
| 99 | A **(01B)** + | A + E | B | 35,75 | 1,605046 | 40 |
| | E/B **(01A+01D)** | | | 32,05 | 2,957877 | 907 |
| 61/8 | A **(11D)** + | A + E | A + B | 26,26 | 4,575963 | 37667 |
| | E/B **(11C)** | | | 27,68 | 4,226493 | 16846 |
| 7344/2 | B **(19D)** + D **(19B)** | B + D | B | 33,59 28,37 | 3,075151 3,588211 | 1189 3874 |
| 8615/1 | B **(20A+20C)**+ E/B **(20D)** | B + E | B | 34 29,01 | 2,954082 3,840392 | 900 6925 |
| 7778B15 | B **(32A)** + F **(32D+32F)** | B + F | B | 36,74 36,96 | 2,144987 1,886284 | 140 77 |

| | | | | | | |
|---|---|---|---|---|---|---|
| ^{a} N₀were calculated using the regression curves presented in Figure 2 | | | | | | |

## Claims

1. An *ex vivo* or *in vitro* method for the identification of the presence of DNA of a genotype of *Chlamydophila psittaci* (*Cp. Psittaci*) in a sample, said method comprising the steps of:
- incubating said sample with a first oligonucleotide which is capable of specifically hybridising to DNA of a genotype of *Cp. psittaci,* incubating said sample with at least one second oligonucleotide, which is a competitor oligonucleotide, comprising a sequence corresponding to a sequence within the DNA of a genotype of *Cp. psittaci* other than the genotype to which the first probe is directed, which can be aligned with the sequence of said first probe, and,
- determining the binding of said first oligonucleotide to DNA within said sample, which binding is indicative of the presence of DNA of a genotype of *Cp. psittaci* in said sample.

2. The method according to claim 1, wherein said first nucleotide comprises a sequence of at least 15 nucleotides of the OmpA gene of one of the *Cp. psittaci* genotypes.

3. The method according to claim 1 or 2, wherein said first nucleotide comprises a sequence of at least 15 nucleotides within the region from nucleotide 450 to nucleotide 600 or from nucleotide 900 to 1100 of the OmpA sequence corresponding to GB accession AF269281 and depicted in SEQ ID NO: 54 .

4. The method of any one of claims 1 to 3, wherein said genotype of *Cp. psittaci* is selected from genotypes A, B, C, D, E, F and EB, wherein genotype EB is **characterised in that** it comprises the OmpA sequence depicted in SEQ ID NO:51.

5. The method according to any one of claims 1 to 4, wherein said first oligonucleotide is labeled with a chromophoric group at its 5' and with a quencher group at its 3' end.

6. The method of any one of claims 1 to 5, wherein said sample is incubated with more than one first oligonucleotide, and wherein each of said more than one first nucleotide is capable of hybridising to DNA of a genotype of *Cp. psittaci.*

7. The method according to any one of claims 1 to 6, wherein the first oligonucleotide comprises a sequence selected from the group consisting of
- sequence corresponding to SEQ ID NO: 1 for genotype A,
- sequence corresponding SEQ ID NO: 2, for genotype B
- sequence corresponding SEQ ID NO: 3 for genotype C,
- sequence corresponding SEQ ID NO: 4, for genotype D;
- sequence corresponding SEQ ID NO: 5, for genotype E;
- sequence corresponding SEQ ID NO: 6, for genotype F and
- sequence corresponding SEQ ID NO: 24, for genotype EB, wherein genotype EB is **characterised in that** it comprises the OmpA sequence depicted in SEQ ID NO:51, or a sequence which differs in one to three nucleotides from the provided sequences, capable of hybridizing specifically to said genotype.

8. The method according to any of claims 1 to 7 , wherein said first and said second oligonucleotide are selected from the group consisting of:
- said second oligonucleotide comprises the sequence of SEQ ID NO: 8, and said first oligonucleotide comprises the sequence of SEQ ID NO: 1;
- said second oligonucleotide comprises the sequence of SEQ ID NO: 7, and said first oligonucleotide comprises the sequence of SEQ ID NO: 2;
- said second oligonucleotide comprises the sequence of SEQ ID NO: 10, and wherein said first oligonucleotide comprises the sequence of SEQ ID NO: 2;
- said second oligonucleotide comprises the sequence of SEQ ID NO: 9, and said first oligonucleotide comprises the sequence of SEQ ID NO: 5;
- said second oligonucleotide comprises the sequence of SEQ ID NO: 11, and said first oligonucleotide comprises the sequence of SEQ ID NO: 5.

9. The method according to any of claims 1 to 8, wherein the binding of said first oligonucleotide is determined by PCR amplification with a forward and a reverse primer.

10. The method according to claim 9, wherein said forward and reverse primer are located about 1 to 100 bp 3' and 5' from said first oligonucleotide.

11. The method according to claim 9 or 10, wherein said forward and reverse primer for said PCR amplification of said first oligonucleotide are selected from the group consisting of
- primers comprising the sequence of SEQ ID NO: 12 and SEQ ID NO: 13, when the first oligonucleotide comprises the sequence of SEQ ID NO: 1;
- primers comprising the sequence of SEQ ID NO: 14 and SEQ ID NO: 15, when the first oligonucleotide comprises the sequence of SEQ ID NO: 2;
- primers comprising the sequence of SEQ ID NO: 16 and SEQ ID NO: 17, when the first oligonucleotide comprises the sequence of SEQ ID NO: 3;
- primers comprising the sequence of SEQ ID NO: 18 and SEQ ID NO: 19, when the first oligonucleotide comprises the sequence of SEQ ID NO: 4;
- primers comprising the sequence of SEQ ID NO: 20 and SEQ ID NO: 21, when the first oligonucleotide comprises the sequence of SEQ ID NO: 5;
- primers comprising the sequence of SEQ ID NO: 22 and SEQ ID NO: 23 when the first oligonucleotide comprises the sequence of SEQ ID NO: 6;
- primers comprising the sequence of SEQ ID NO: 25 and SEQ ID NO: 26 when the first oligonucleotide comprises the sequence of SEQ ID NO: 24;
or primers which have a sequence differing at maximally 5 nucleotides from said primers without affecting their ability to function as a primer for PCR amplification.

12. The method according to any of claims 1 to 11, wherein said sample is from a bird.

13. The method according to claim 12, wherein said bird is in the stage of development in which the maternal immunity of said bird disappears.

14. The method according to claim 13, wherein said bird is a duck of about 6 weeks after hatching.

15. The method according to any one of claims 6 to 14, which is a method for specifically identifying the Cp. psittaci genotype present in the sample.

16. The method according to any of the claims 1 to 15, for determining the efficacy of a treatment against a *Cp. psittaci* infection.

17. A diagnostic kit for the identification of the presence of DNA of a genotype of *Chlamydophila psittaci* (*Cp. Psittaci*) in a sample, comprising the genotype specific probes and competitor probes:
- genotype-specific oligonucleotide comprising the sequence of SEQ ID NO: 1, and genotype specific competitor oligonucleotide comprising the sequence of SEQ ID NO: 8;
- genotype-specific oligonucleotide comprising the sequence of SEQ ID NO: 2, and genotype specific competitor oligonucleotide comprising the sequence of SEQ ID NO: 7;
- genotype-specific oligonucleotide comprising the sequence of SEQ ID NO: 2, and genotype specific competitor oligonucleotide comprising the sequence of SEQ ID NO: 10;
- genotype-specific oligonucleotide comprising the sequence of SEQ ID NO: 5, and genotype specific competitor oligonucleotide comprising the sequence of SEQ ID NO: 9; and
- genotype-specific oligonucleotide comprising the sequence of SEQ ID NO: 5, and genotype specific competitor said first oligonucleotide comprises the sequence of SEQ ID NO: 11.

18. The diagnostic kit of claim 17, comprising one or more of the oligonucleotides selected from the group consisting of SEQ ID NO: 3, SEQ ID NO: 4, SEQ ID NO: 6, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22 and SEQ ID NO: 23.

19. Use of genotype specific probes in combination with competitors probes for the *ex vivo* or *in vitro* identification of a genotype of *Cp. psittaci,* wherein said competitor probe comprises a sequence corresponding to a sequence within the DNA of a genotype of *Cp. psittaci* other than the genotype to which the genotype specific probe is directed, which can be aligned with the sequence of said genotype specific probe.

20. The use according to claim 19, wherein the identification of said genotype is for diagnosis and treatment-follow-up of *Cp. psittaci* infections from respiratory samples of humans.

## Patentansprüche

1. Ex-vivo- oder In-vitro-Verfahren zur Feststellung des Vorhandenseins von DNA eines Genotyps von Chlamydophila psittaci (Cp. psittaci), wobei das Verfahren folgende Schritte umfasst:
- Inkubieren der Probe mit einem ersten Oligonukleotid, das imstande ist, spezifisch an DNA eines Genotyps von Cp. psittaci zu hybridisieren, Inkubieren der Probe mit mindestens einem zweiten Oligonukleotid, das ein Kompetitor-Oligonukleotid ist, umfassend eine Sequenz, die einer Sequenz innerhalb der DNA eines Genotyps von Cp. psittaci entspricht, der anders als der Genotyp ist, auf den die erste Sonde gerichtet ist, die mit der Sequenz der ersten Sonde ausgerichtet sein kann, und
- Bestimmen der Bindung des ersten Oligonukleotids an DNA innerhalb der Probe, wobei die Bindung das Vorhandensein von DNA eines Genotyps Cp. psittaci in der Probe anzeigt.

2. Verfahren nach Anspruch 1, wobei das erste Nukleotid eine Sequenz von mindestens 15 Nukleotiden des OmpA-Gens eines der Cp. psittaci Genotypen umfasst.

3. Verfahren nach Anspruch 1 oder 2, wobei das erste Nukleotid eine Sequenz von mindestens 15 Nukleotiden innerhalb der Region von Nukleotid 450 bis Nukleotid 600 oder von Nukleotid 900 bis 1100 der OmpA-Sequenz umfasst, die GB-Zugang AF269281 entspricht und in SEQ ID NR: 54 dargestellt ist.

4. Verfahren nach einem der Ansprüche 1 bis 3, wobei der Genotyp von Cp. psittaci ausgewählt ist aus Genotypen A, B, C, D, E, F und EB, wobei Genotyp EB **dadurch gekennzeichnet ist, dass** er die in SEQ ID NR: 51 dargestellte OmpA-Sequenz umfasst.

5. Verfahren nach einem der Ansprüche 1 bis 4, wobei das erste Oligonukleotid mit einer chromophoren Gruppe an seinem 5'- und einer Quencher-Gruppe an seinem 3'-Ende markiert ist.

6. Verfahren nach einem der Ansprüche 1 bis 5, wobei die Probe mit mehrer als einem ersten Oligonukleotid markiert ist und wobei jedes der mehr als ein erstes Nukleotide imstande ist, an DNA eines Genotyps von Cp. psittaci zu hybridisieren.

7. Verfahren nach einem der Ansprüche 1 bis 6, wobei das erste Oligonukleotid eine Sequenz umfasst, die ausgewählt ist aus der Gruppe bestehend aus
- Sequenz entsprechend SEQ ID NR: 1 für Genotyp A,
- Sequenz entsprechend SEQ ID NR: 2 für Genotyp B,
- Sequenz entsprechend SEQ ID NR: 3 für Genotyp C,
- Sequenz entsprechend SEQ ID NR: 4 für Genotyp D,
- Sequenz entsprechend SEQ ID NR: 5 für Genotyp E,
- Sequenz entsprechend SEQ ID NR: 6 für Genotyp F und
- Sequenz entsprechend SEQ ID NR: 24 für Genotyp EB, wobei Genotyp EB **dadurch gekennzeichnet ist, dass** er die in SEQ ID NR: 51 dargestellte OmpA-Sequenz umfasst oder eine Sequenz, die sich in einem bis drei Nukleotiden von den vorgesehenen Sequenzen unterscheidet, die imstande ist, spezifisch an den Genotyp zu hybridisieren.

8. Verfahren nach einem der Ansprüche 1 bis 7, wobei das erste und das zweite Oligonukleotid ausgewählt sind aus der Gruppe bestehend aus:
- dem zweiten Oligonukleotid, umfassend die Sequenz von SEQ ID NR: 8, und dem ersten Oligonukleotid, umfassend die Sequenz von SEQ ID NR: 1;
- dem zweiten Oligonukleotid, umfassend die Sequenz von SEQ ID NR: 7, und dem ersten Oligonukleotid, umfassend die Sequenz von SEQ ID NR: 2;
- dem zweiten Oligonukleotid, umfassend die Sequenz von SEQ ID NR: 10, und wobei das erste Oligonukleotid die Sequenz von SEQ ID NR: 2 umfasst;
- dem zweiten Oligonukleotid, umfassend die Sequenz von SEQ ID NR: 9, und dem ersten Oligonukleotid, umfassend die Sequenz von SEQ ID NR: 5;
- dem zweiten Oligonukleotid, umfassend die Sequenz von SEQ ID NR: 11, und dem ersten Oligonukleotid, umfassend die Sequenz von SEQ ID NR: 5.

9. Verfahren nach einem der Ansprüche 1 bis 8, wobei die Bindung des ersten Oligonukleotids durch PCR-Amplifikation mit einem Vorwärts- und einem Rückwärts-Primer bestimmt wird.

10. Verfahren nach Anspruch 9, wobei der Vorwärts- und Rückwärts-Primer etwa 1 bis 100 bp 3' und 5' vom ersten Oligonukleotid angeordnet sind.

11. Verfahren nach Anspruch 9 oder 10, wobei der Vorwärts- und Rückwärts-Primer für die PCR-Amplifikation des ersten Oligonukleotids ausgewählt sind aus der Gruppe bestehend aus
- Primern, die die Sequenz von SEQ ID NR: 12 und SEQ ID NR: 13 umfassen, wenn das erste Oligonukleotid die SEQ ID NR: 1 umfasst;
- Primern, die die Sequenz von SEQ ID NR: 14 und SEQ ID NR: 15 umfassen, wenn das erste Oligonukleotid die SEQ ID NR: 2 umfasst;
- Primern, die die Sequenz von SEQ ID NR: 16 und SEQ ID NR: 17 umfassen, wenn das erste Oligonukleotid die SEQ ID NR: 3 umfasst;
- Primern, die die Sequenz von SEQ ID NR: 18 und SEQ ID NR: 19 umfassen, wenn das erste Oligonukleotid die SEQ ID NR: 4 umfasst;
- Primern, die die Sequenz von SEQ ID NR: 20 und SEQ ID NR: 21 umfassen, wenn das erste Oligonukleotid die SEQ ID NR: 5 umfasst;
- Primern, die die Sequenz von SEQ ID NR: 22 und SEQ ID NR: 23 umfassen, wenn das erste Oligonukleotid die SEQ ID NR: 6 umfasst;
- Primern, die die Sequenz von SEQ ID NR: 25 und SEQ ID NR: 26 umfassen, wenn das erste Oligonukleotid die SEQ ID NR: 24 umfasst;
oder Primern, die eine Sequenz haben, die sich bei maximal 5 Nukleotiden von den Primern unterscheidet, ohne deren Fähigkeit zu beeinträchtigen, als Primer zur PCR-Amplifikation zu dienen.

12. Verfahren nach einem der Ansprüche 1 bis 11, wobei die Probe von einem Vogel stammt.

13. Verfahren nach Anspruch 12, wobei sich der Vogel in dem Entwicklungsstadium befindet, in dem die Leihimmunität des Vogels verschwindet.

14. Verfahren nach Anspruch 13, wobei der Vogel eine Ente etwa 6 Wochen nach dem Schlüpfen ist.

15. Verfahren nach einem der Ansprüche 6 bis 14, das ein Verfahren zum spezifischen Identifizieren des Cp. psittaci Genotyps ist, der in der Probe vorhanden ist.

16. Verfahren nach einem der Ansprüche 1 bis 15, zum Bestimmen der YJirksamkeit einer Behandlung gegen eine Cp. psittaci Infektion.

17. Diagnostisches Kit zur Feststellung des Vorhandenseins von DNA eines Genotyps von Chlamydophila psittaci (Cp. psittaci) in einer Probe, umfassend die Genotyp-spezifischen Sonden und Kompetitor-Sonden:
- Genotyp-spezifisches Oligonukleotid, umfassend die Sequenz von SEQ ID NR: 1, und Genotyp-spezifisches Kompetitor-Oligonukleotid, umfassend die Sequenz von SEQ ID NR: 8;
- Genotyp-spezifisches Oligonukleotid, umfassend die Sequenz von SEQ ID NR: 2, und Genotyp-spezifisches Kompetitor-Oligonukleotid, umfassend die Sequenz von SEQ ID NR: 7;
- Genotyp-spezifisches Oligonukleotid, umfassend die Sequenz von SEQ ID NR: 2, und Genotyp-spezifisches Kompetitor-Oligonukleotid, umfassend die Sequenz von SEQ ID NR: 10;
- Genotyp-spezifisches Oligonukleotid, umfassend die Sequenz von SEQ ID NR: 5 und Genotyp-spezifisches Kompetitor-Oligonukleotid, umfassend die Sequenz von SEQ ID NR: 9; und
- Genotyp-spezifisches Oligonukleotid, umfassend die Sequenz von SEQ ID NR: 5 und Genotyp-spezifisches Kompetitor-Oligonukleotid, umfassend die Sequenz von SEQ ID NR: 11.

18. Diagnostisches Kit nach Anspruch 17, umfassend eines oder mehrere der Oligonukleotide, ausgewählt aus der Gruppe bestehend aus SEQ ID NR: 3, SEQ ID NR: 4, SEQ ID NR: 6, SEQ ID NR: 12, SEQ ID NR: 13, SEQ ID NR: 14, SEQ ID NR: 15, SEQ ID NR: 16, SEQ ID NR: 17, SEQ ID NR: 18, SEQ ID NR: 19, SEQ ID NR: 20, SEQ ID NR: 21, SEQ ID NR: 22 und SEQ ID NR: 23.

19. Verwendung Genotyp-spezifischer Sonden in Kombination mit Kompetitor-Sonden für die Ex-vivo- oder In-vitro-Identifizierung eines Genotyps von Cp. psittaci, wobei die Kompetitor-Sonde eine Sequenz umfasst, die einer Sequenz innerhalb der DNA eines Genotyps von Cp. psittaci entspricht, der nicht der Genotyp ist, auf den die Genotyp-spezifische Sonde gerichtet ist, die mit der Sequenz der Genotyp-spezifischen Sonde ausgerichtet sein kann.

20. Verwendung nach Anspruch 19, wobei die Identifizierung des Genotyps der Diagnose und Behandlungsnachkontrolle von Cp. psittaci Infektionen aus Atemproben von Menschen dient.

## Revendications

1. Procédé *ex vivo* ou *in vitro* pour l'identification de la présence d'ADN d'un génotype de *Chlamydophila psittaci* (*Cp. Psittaci*) dans un échantillon, ledit procédé comprenant les étapes de :
- incubation dudit échantillon avec un premier oligonucléotide qui est susceptible de s'hybrider de manière spécifique à l'ADN d'un génotype de *Cp. psittaci,* incubation dudit échantillon avec au moins un second oligonucléotide, qui est un oligonucléotide concurrent, comprenant une séquence correspondant à une séquence à l'intérieur de l'ADN d'un génotype de *Cp. psittaci* autre que le génotype vers lequel la première sonde est dirigée, qui peut être alignée avec la séquence de ladite première sonde, et
- détermination de la liaison dudit premier oligonucléotide à l'ADN à l'intérieur dudit échantillon, laquelle liaison est indicative de la présence d'ADN d'un génotype de *Cp. psittaci* dans ledit échantillon.

2. Procédé selon la revendication 1, dans lequel ledit premier nucléotide comprend une séquence d'au moins 15 nucléotides du gène OmpA d'un des génotypes de *Cp. psittaci.*

3. Procédé selon la revendication 1 ou 2, dans lequel ledit premier nucléotide comprend une séquence d'au moins 15 nucléotides à l'intérieur de la région allant du nucléotide 450 au nucléotide 600 ou allant des nucléotides 900 à 1100 de la séquence OmpA correspondant au matricule GB AF269281 et décrit dans SEQ ID n° 54.

4. Procédé selon l'une quelconque des revendications 1 à 3, dans lequel ledit génotype de *Cp. psittaci* est sélectionné à partir de génotypes A, B, C, D, E, F et EB, dans lesquels le génotype EB est **caractérisé en ce qu'**il comprend la séquence OmpA décrite dans SEQ ID n° 51.

5. Procédé selon l'une quelconque des revendications 1 à 4, dans lequel ledit premier oligonucléotide est étiqueté avec un groupement chromophore au niveau de son extrémité 5' et avec un groupe désactiveur au niveau de son extrémité 3'.

6. Procédé selon l'une quelconque des revendications 1 à 5, dans lequel ledit échantillon est incubé avec plus d'un premier oligonucléotide, et dans lequel chacun desdits plus d'un premier nucléotide est susceptible de s'hybrider à l'ADN d'un génotype de *Cp. psittaci.*

7. Procédé selon l'une quelconque des revendications 1 à 6, dans lequel le premier oligonucléotide comprend une séquence sélectionnée à partir du groupe constitué par
- une séquence correspondant à SEQ ID n° 1 pour le génotype A,
- une séquence correspondant à SEQ ID n° 2, pour le génotype B
- une séquence correspondant à SEQ ID n° 3 pour le génotype C,
- une séquence correspondant à SEQ ID n° 4, pour le génotype D ;
- une séquence correspondant à SEQ ID n° 5, pour le génotype E ;
- une séquence correspondant à SEQ ID n° 6, pour le génotype F, et
- une séquence correspondant à SEQ ID n° 24, pour le génotype EB, dans lequel le génotype EB est **caractérisé en ce qu'**il comprend la séquence OmpA décrite dans SEQ ID n° 51, ou une séquence qui diffère dans un à trois nucléotides des séquences fournies, susceptible de s'hybrider de manière spécifique audit génotype.

8. Procédé selon l'une quelconque des revendications 1 à 7, dans lequel ledit premier et ledit second oligonucléotide sont sélectionnés à partir du groupe constitué par :
- ledit second oligonucléotide qui comprend la séquence de SEQ ID n° 8, et ledit premier oligonucléotide qui comprend la séquence de SEQ ID n° 1 ;
- ledit second oligonucléotide qui comprend la séquence de SEQ ID n° 7, et ledit premier oligonucléotide qui comprend la séquence de SEQ ID n° 2 ;
- ledit second oligonucléotide qui comprend la séquence de SEQ ID n° 10, et dans lequel ledit premier oligonucléotide comprend la séquence de SEQ ID n° 2 ;
- ledit second oligonucléotide qui comprend la séquence de SEQ ID n° 9, et ledit premier oligonucléotide qui comprend la séquence de SEQ ID n° 5 ;
- ledit second oligonucléotide qui comprend la séquence de SEQ ID n° 11 et ledit premier oligonucléotide qui comprend la séquence de SEQ ID n° 5.

9. Procédé selon l'une quelconque des revendications 1 à 8, dans lequel la liaison dudit premier oligonucléotide est déterminée par amplification PCR avec une amorce sens et une amorce antisens.

10. Procédé selon la revendication 9, dans lequel lesdites amorces sens et antisens sont situées à environ 1 à 100 bp 3' et 5' dudit premier oligonucléotide.

11. Procédé selon la revendication 9 ou 10, dans lequel lesdites amorces sens et antisens pour ladite amplification PCR dudit premier oligonucléotide sont choisies à partir du groupe constitué par
- des amorces comprenant la séquence de SEQ ID n° 12 et SEQ ID n° 13, quand le premier oligonucléotide comprend la séquence de SEQ ID n° 1 ;
- des amorces comprenant la séquence de SEQ ID n° 14 et SEQ ID n° 15, quand le premier oligonucléotide comprend la séquence de SEQ ID n° 2 ;
- des amorces comprenant la séquence de SEQ ID n° 16 et SEQ ID n° 17, quand le premier oligonucléotide comprend la séquence de SEQ ID n° 3 ;
- des amorces comprenant la séquence de SEQ ID n° 18 et SEQ ID n° 19, quand le premier oligonucléotide comprend la séquence de SEQ ID n° 4 ;
- des amorces comprenant la séquence de SEQ ID n° 20 et SEQ ID n° 21, quand le premier oligonucléotide comprend la séquence de SEQ ID n° 5 ;
- des amorces comprenant la séquence de SEQ ID n° 22 et SEQ ID n° 23 quand le premier oligonucléotide comprend la séquence de SEQ ID n° 6 ;
- des amorces comprenant la séquence de SEQ ID n° 25 et SEQ ID n° 26 quand le premier oligonucléotide comprend la séquence de SEQ ID n° 24 ;
ou des amorces qui ont une séquence différant au maximum de 5 nucléotides desdites amorces sans affecter leur capacité à fonctionner comme amorce pour l'amplification PCR.

12. Procédé selon l'une quelconque des revendications 1 à 11, dans lequel ledit échantillon provient d'un oiseau.

13. Procédé selon la revendication 12, dans lequel ledit oiseau est au stade de développement dans lequel l'immunité maternelle dudit oiseau disparaît.

14. Procédé selon la revendication 13, dans lequel ledit oiseau est un canard d'environ 6 semaines après éclosion.

15. Procédé selon l'une quelconque des revendications 6 à 14, qui est un procédé pour identifier de manière spécifique le génotype de *Cp. psittaci* présent dans l'échantillon.

16. Procédé selon l'une quelconque des revendications 1 à 15, pour déterminer l'efficacité d'un traitement contre une infection par *Cp. psittaci.*

17. Kit de diagnostic pour l'identification de la présence d'ADN d'un génotype de *Chlamydophila psittaci* (*Cp. psittaci*) dans un échantillon, comprenant les sondes spécifiques au génotype et des sondes de concurrents :
- un oligonucléotide spécifique au génotype comprenant la séquence de SEQ ID n° 1, et un oligonucléotide concurrent spécifique au génotype comprenant la séquence de SEQ ID n° 8 ;
- un oligonucléotide spécifique au génotype comprenant la séquence de SEQ ID n° 2, et un oligonucléotide concurrent spécifique au génotype comprenant la séquence de SEQ ID n° 7 ;
- un oligonucléotide spécifique au génotype comprenant la séquence de SEQ ID n° 2, et un oligonucléotide concurrent spécifique au génotype comprenant la séquence de SEQ ID n° 10 ;
- un oligonucléotide spécifique au génotype comprenant la séquence de SEQ ID n° 5, et un oligonucléotide concurrent spécifique au génotype comprenant la séquence de SEQ ID n° 9 ; et
- un oligonucléotide spécifique au génotype comprenant la séquence de SEQ ID n° 5, et un oligonucléotide concurrent spécifique au génotype comprenant la séquence de SEQ ID n° 11.

18. Kit de diagnostic selon la revendication 17, comprenant un ou plusieurs des oligonucléotides sélectionnés à partir du groupe constitué par SEQ ID n° 3, SEQ ID n° 4, SEQ ID n° 6, SEQ ID n° 12, SEQ ID n° 13, SEQ ID n° 14, SEQ ID n° 15, SEQ ID n° 16, SEQ ID n° 17, SEQ ID n° 18, SEQ ID n° 19, SEQ ID n° 20, SEQ ID n° 21, SEQ ID n° 22 et SEQ ID n° 23.

19. Utilisation de sondes spécifiques au génotype en combinaison avec des sondes de concurrents pour l'identification *ex vivo* ou *in vitro* d'un génotype de *Cp. psittaci,* dans laquelle ladite sonde de concurrent comprend une séquence correspondant à une séquence à l'intérieur de l'ADN d'un génotype de *Cp. psittaci* autre que le génotype vers lequel la sonde spécifique au génotype est dirigée, qui peut être alignée avec la séquence de ladite sonde spécifique au génotype.

20. Utilisation selon la revendication 19, dans laquelle l'identification dudit génotype sert au diagnostic et au suivi de traitement d'infections par *Cp. psittaci* à partir d'échantillons respiratoires d'humains.
